# EUROPEAN PATENT SPECIFICATION

(11) **EP 1 589 964 B1**
(45) Date of publication and mention of the grant of the patent: **23.11.2011**
(21) Application number: 04702609.1
(22) Date of filing: 16.01.2004
(51) Int. Cl.: A61K 31/355, A61K 31/661, A61K 31/6615, A61P 25/28, A61P 3/10, A61P 9/10

(54) **COMPOUNDS HAVING ANTI-PROLIFERATIVE PROPERTIES**
VERBINDUNGEN MIT PROLIFERATIONSHEMMENDEN EIGENSCHAFTEN
COMPOSES POSSEDANT DES PROPRIETES ANTIPROLIFERATIVES

(30) Priority: 17.01.2003 AU 2003900200; 09.04.2003 AU 2003901698
(43) Date of publication of application: 02.11.2005
(73) Proprietor: Vital Health Sciences Pty Ltd., Melbourne, VIC 3000 (AU)
(72) Inventor: WEST, Simon, Michael, Williamstown, VIC 3016 (AU); OGRU, Esra, Glen Waverley VIC 3150 (AU)
(74) Representative: Vossius & Partner
(86) International application number: PCT/AU2004/000056
(87) International publication number: WO 2004/064831

(56) References cited:
- EP-A- 0 669 132
- EP-A1- 0 699 437
- EP-A1- 1 053 749
- EP-A2- 0 324 387
- EP-A2- 0 436 911
- WO-A-00/44237
- WO-A-00/44375
- WO-A-01/13901
- WO-A-02/26238
- WO-A1-00/57876
- WO-A1-01/22937
- WO-A1-02/36736
- WO-A1-03/026673
- US-A- 6 143 770
- US-A1- 2001 044 462
- DEVARAJ S. ET AL: 'Modulation of Monocyte-Macrophage Function with alfa-Tocopherol: Implications for Atherosclerosis.' NUTRITION REVIEWS vol. 60, no. 1, January 2002, pages 8 - 14, XP008087151

## Description

### Field of the invention

The invention relates to the ability of modified electron transfer agents to inhibit plaque formation in the vascular system.

### Background of the invention

In this specification, where a document, act or item of knowledge is referred to or discussed, this reference or discussion is not an admission that the document, act or item of knowledge or any combination thereof was at the priority date part of common general knowledge; or known to be relevant to an attempt to solve any problem with which this specification is concerned.

Atherosclerosis is a disease of the arterial intima leading to formation of fibrous (atheromatous) plaques and to stenosis or occlusion of the lumen. Arteries affected with atherosclerosis lose their elasticity, and as atheromas grow, the arteries narrow and, with time, may rupture. Blood then enters the atheroma, making it larger, so that it narrows the artery even more. A ruptured atheroma can spill its fatty contents and trigger the formation of a blood clot (thrombus) that further narrows or detaches sometimes causing an occlusion (embolism). Atherosclerosis can affect the arteries of the brain, heart, kidneys, other vital organs, and the arms and legs. When an embolism develops in the arteries that supply the brain (carotid arteries), a stroke may occur, and when it develops in the arteries that supply the heart (coronary arteries), a heart attack may occur.

Risk factors include, but are not limited to ageing, high blood pressure, cigarette smoking, obesity, diabetes, reduced circulating high density lipoprotein (HDL) levels, elevated lipoprotein particle [Lp(a)] levels, elevated oxidized low density lipoprotein (LDL) levels, iatrogenically induced increases in levels of oxidised LDL, and lack of exercise, as all increase the likelihood of physical injury to the intima and atherogenesis.

Many scientists currently believe atherosclerosis begins because the innermost layer of the artery, the endothelium, becomes injured. As with any other part of the body which is injured, the artery then becomes inflammed. This inflammation causes the following natural processes:
- proliferation of monocytes which mature into macrophages;
- expression of scavenger receptors such as CD36 receptors on monocytes/macrophages; and
- proliferation of the smooth muscle cells (SMCs) to repair the injury.

At the same time, there is an accumulation of these and other molecules (lymphocytes, oxidized low-density lipoprotein (LDL), fibrin, platelets, cellular debris and calcium) through the damaged endothelium into the intima of the arterial wall. This accumulation stimulates further inflammatory mediators that modify mRNA expression of signalling proteins (protein kinase C-α (PKC-α), VCAM, etc). This inflammation leads to further accumulation of the above molecules in the intima and growth of the plaque,

Monocytes are a type of white blood cell. Monocytes mature into macrophages when they pass into tissue and it is the macrophages which are the operational white blood cells. Macrophages take up and kill disease causing microorganisms and remove damaged cells. The macrophages which have accumulated recruit the assistance of further monocytes and attempt to remove the accumulating oxidized LDL. The CD36 receptors on the macrophage cell surface participate by adhering to the oxidized LDL molecules. When this removal process is unregulated, foam cells are formed. These foam cells also accumulate in the plaque.

CD36 receptors are a variety of cell surface glycoprotein and known to be part of a larger group of scavenger receptors including SR-A, MARCO, CD68, LOX-1 and SR-BI. Scavenger receptors, including CD36 receptors, are thought to be important during macrophage uptake of oxidized LDL and foam cell formation. CD36 receptors are known to contribute to uptake of modified lipoproteins and act as receptors for thrombospondin, type I & IV collagens, fatty acids and polyanionic phospholipids.

If the proliferation of smooth muscle cells becomes excessive due to the continued inflammatory response to the growing plaque, these smooth muscle cells also contribute to the ongoing plaque formation.

Increased levels of oxidized LDLs are also thought to cause inflammation of the arterial wall leading to the above responses and the formation of atherosclerotic plaque.

As a result, substances which inhibit smooth muscle cell proliferation, inhibit monocyte proliferation, reduce uptake of oxidised LDL or inhibit the activity of scavenger receptors may be useful in treatment of atherosclerosis.

### Symptoms & Treatment

There are no current direct treatments for the symptoms associated with atherosclerosis. Health professionals therefore aim to eliminate controllable risk factors, such as high blood cholesterol levels. Over recent years dieticians have also encouraged high risk individuals to consider a broad variety of protective foods containing various phytochemicals and antioxidant nutrients such as vitamin E.

Since most atherogenic serum cholesterol is carried in the LDL fraction, reduction of elevated LDL levels is the principle clinical means of treating atherosclerosis. This, however, is an indirect means of treating atherosclerotic disease processes because it does not directly stop initiation. Currently, there are no effective drugs available to directly treat and reduce formation of atherosclerotic plaques.

Hyperlipidaemic compounds indirectly inhibit aortic (artery which leads to the heart) wall cell proliferation to a limited extent, but long-term treatment is required to have any effect. Removal of large amounts of cholesterol over longer periods has its own risks. Cholesterol is a substrate for synthesis of many important compounds including steroid hormones, vitamin D, ubiquinone, bile acids, dolichol, farnesylated proteins, haem A and tRNA. So aggressive cholesterol removal may be associated with problems in some individuals. Again, hyperlipidaemic compounds do not directly treat the fundamental causes of atherosclerosis such as oxidatively modified LDL and excessive smooth muscle cell proliferation and thus are not ideal options. Some compounds such as anti-cancer drugs will inhibit excessive smooth cell proliferation, but these cause severe side effects and are therefore not a valid option.

One experimental drug is being clinically studied and thought to act by reducing the amount of VCAM-1 proteins which reduces the uptake of white blood cells (monocytes/macrophages) at sites of inflammation.

There are no drugs that are known to directly modify expression of CD36 receptors or other scavenger receptors to treat atherosclerosis.

Currently, there are no effective options available to directly treat excessive smooth muscle cell proliferation.

### Tocopherol

Low levels of α-tocopherol (vitamin E) have been associated with increased incidence of coronary heart disease. Conversely, increased intake of α-tocopherol has been shown to have protective effects against heart disease. Since vitamin E is an antioxidant, it is thought to target the cause of atherosclerosis by preventing oxidation of LDL. Studies have also been undertaken to examine potential non-antioxidant mechanisms of vitamin E which could prevent formation of atherosclerotic plaques. Such responses include inhibition of smooth muscle cell proliferation, preservation of endothelial function, inhibition of monocyte-endothelial adhesion, inhibition of monocyte reactive oxygen species and cytokine release, and inhibition of platelet adhesion and aggregation.

Clinical trials with vitamin E have however been equivocal in demonstrating treatment of atherosclerosis. Current vitamin E supplements are therefore not a useful clinical option to combat atherosclerosis.

### Other diseases and conditions

There are other diseases and conditions where proliferation of monocytes/macrophages, proliferation of smooth muscle cells, scavenger receptor expression or uptake of oxidized LDL are a problem. Examples of other diseases include Alzheimer's disease and diabetes. An example of a related conditions is the increased levels of oxidized LDL caused by certain drugs (iatrogenic diseases) such as ritonovir.

There is a need for a therapy with minimal side effects and low dosage which will assist to reduce one or more of proliferation of monocytes/macrophages, proliferation of smooth muscle cells, scavenger receptor expression or uptake of oxidized LDL.

### <Summary of the Invention>

It has now been found that the phosphate derivatives of electron transfer agents are more effective than the non-phosphorylated electron transfer agents at inhibiting the occurrence of one or more of the following conditions:
- smooth muscle cell proliferation;
- monocyte/macrophage proliferation;
- scavenger receptor expression; or
- oxidised LDL uptake.

The proliferation of smooth muscle cells or monocytes/macrophages may be slowed or prevented altogether and the scavenger cell expression or oxidised LDL uptake may be reduced or restrained by the phosphate derivatives of electron transfer agents in a dose responsive manner.

In one embodiment, the present invention relates to a pharmaceutical composition for use in inhibiting plaque formation in the vascular system, wherein the composition comprises an effective amount of one or more phosphate derivatives of one or more electron transfer agents selected from the group consisting of mono-tocopheryl phosphate, di-tocopheryl phosphate and mixtures thereof.

In a second embodiment a dose form comprising the pharmaceutical composition of the present invention is disclosed.

A further embodiment of the present invention is the use of an effective amount of one or more phosphate derivates of one or more electron transfer agents, selected from the group consisting of mono-tocopheryl phosphate, di-tocopheryl phosphate and mixtures thereof, together with a suitable carrier or diluent in the manufacture of a medicament for inhibiting plaque formation in the vascular system.

Preferably, the subject is an animal, more preferably the animal is a human.

The term "effective amount" is used herein to refer to an amount which is sufficient to inhibit plaque formation in the vascular system.

A person skilled in the art will understand that this amount will vary from patient to patient.

Typically, an effective amount of one or more phosphate derivatives of one or more electron transfer agents is an amount which is 0.1 to 10 times the average α-tocopherol plasma or tissue N levels (average α-tocopherol plasma concentration is between 30-50µM). More preferably, the effective amount is an amount which is 2 to 3 times the average α-tocopherol plasma or tissue levels.

The typical treatment would involve administering to a subject between 50 to 1000 mg of one or more phosphate derivatives of electron transfer agents per day until the average plasma/tissue concentrations of the electron transfer agent is between 2 to 10 times the average plasma concentration of α-tocopherol. Intake of one or more phosphate derivatives of electron transfer agents would then be adjusted to maintain the desired plasma/tissue concentration.

The term "electron transfer agents" is used herein to refer to the class of chemicals which may be phosphorylated and which (in the non-phosphorylated form) can accept an electron to generate a relatively stable molecular radical or accept two electrons to allow the compound to participate in a reversible redox system. Examples of classes of electron transfer agent compounds that may be phosphorylated include hydroxy chromans including alpha-, beta-, gamma- and delta- tocols in enantiomeric and raecemic forms; quinols being the reduced s X of electron transfer agent K1 and ubiquinone; hydroxy carotenoids including retinol; calciferol and ascorbic acid. More specific examples of electron transfer agents are tocopherol and other tocols, retinol, electron transfer agent K1 and mixtures thereof.

The tocols include all isomers of derivatives of 6:hydoxy 2:methyl chroman (see structure below) where R₁, R₂ and R₃ may be hydrogen or methyl groups, that is, the α-5:7:8 tri-methyl; β-5:8 di-methyl; γ-7:8 di-methyl; and δ-8 methyl derivatives. In the tocopherols, R₄ is substituted by 4:8:I2 tri-methyl tridecane and the 2, 4, and 8 positions (see *) may be stereoisomers with R or S activity or be racemic. In the tocotrienols, R₄ is substituted by 4:8:12 tri-methyl trideca-3:7:11 triene and the 2 position may be stereoactive as R or S stereoisomers or be racemic. In the present invention the electron transfer agent is selected from the group consisting of mono-tocopheryl phosphate, di-tocopheryl phosphate and mixtures thereof. Preferably, the electron transfer agent is α-tocopherol.

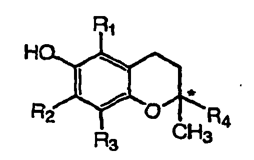

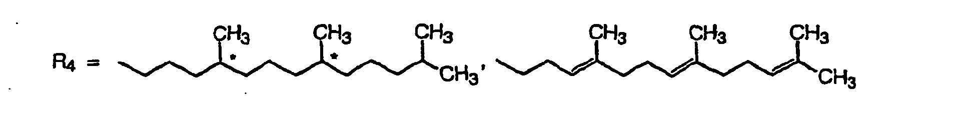

The term "phosphate derivatives" is used herein to refer to the acid forms of phosphorylated electron transfer agents, salts of the phosphates including metal salts such as sodium, magnesium, potassium and calcium and any other derivative where the phosphate proton is replaced by other substituents such as ethyl or methyl groups or phosphatidyl groups. The term includes mixtures of phosphate derivatives, especially those which result from phosphorylation reactions, as well as each of the phosphate derivatives alone. For example, the term includes a mixture of mono-tocopheryl phosphate (TP) and di-tocopheryl phosphate (T2P) as well as each of TP and T2P alone. Suitable mixtures are described in international patent application no. PCT/AU01/01475.

The one or more phosphate derivatives of one or more electron transfer agents is selected from the group consisting of mono-tocopheryl phosphate, di-tocopheryl phosphate and mixtures thereof. Most preferably, the one or more phosphate derivatives of one or more electron transfer agents is a mixture of mono-tocopheryl phosphate and di-tocopheryl phosphate.

In some situations, it may be necessary to use a phosphate derivative such as a phosphatide where additional properties such as increased water solubility are preferred. Phosphatidyl derivatives are amino alkyl derivatives of organic phosphates. These derivatives may be prepared from amines having a structure of R₁R₂N(CH₂)ₙOH wherein n is an integer between 1 and 6 and R₁ and R₂ may be either H or short alkyl chains with 3 or less carbons. R₁ and R₂ may be the same or different. The phosphatidyl derivatives are prepared by displacing the hydroxyl proton of the electron transfer agent with a phosphate entity that is then reacted with an amine, such as ethanolamine or N,N'-dimethylethanolamine, to generate the phosphatidyl derivative of the electron transfer agent. One method of preparation of the phosphatidyl derivatives uses a basic solvent such as pyridine or triethylamine with phosphorous oxychloride to prepare the intermediate which is then reacted with the hydroxy group of the amine to produce the corresponding phosphatidyl derivative, such as P-cholyl-P- tocopheryl dihydrogen phosphate.

In some situations, complexes of phosphate derivatives of the electron transfer agents may also be utilized where additional properties such as improved stability or deliverability may be useful. The term "complexes of phosphate derivatives" refers to the reaction product of one or more phosphate derivatives of electron transfer agents with one or more complexing agents selected from the group consisting of amphoteric surfactants, cationic surfactants, amino acids having nitrogen functional groups and proteins rich in these amino acids as disclosed in international patent application no PCT/AU01/01476, incorporated herein by reference.

The preferred complexing agents are selected from the group consisting of arginine, lysine and tertiary substituted amines, such as those according to the following formula:

NR¹R²R³

wherein R¹ is chosen from the group comprising straight or branched chain mixed alkyl radicals from C6 to C22 and carbonyl derivatives thereof.
R² and R³ are chosen independently from the group comprising H, CH₂COOX, CH₂CHOHCH₂SO₃X, CH₂CHOHCH₂OPO₃X, CH₂CH₂COOX, CH₂COOX, CH₂CH₂CHOHCH₂SO₃X or CH₂CH₂CHOHCH₂OPO₃X and X is H, Na, K or alkanolamine provided R² and R³ are not both H; and
wherein when R¹ is RCO then R² may be CH₃ and R³ may be (CH₂CH₂)N(C₂H₄OH)-H₂CHOPO₃ or R² and R³ together may be N(CH₂)₂N(C₂H₄OH)CH₂COO-.

Preferred complexing agents include arginine, lysine or lauryliminodipropionic acid where complexation occurs between the alkaline nitrogen centre and the phosphoric acid ester to form a stable complex.

The phosphate derivative of the electron transfer agent may be administered to humans or animals through a variety of dose forms such as supplements, enteral feeds, parenteral dose forms, suppositories, nasal delivery forms, dermal delivery including patches and creams.

For example, the phosphate derivative of the electron transfer agent may be administered by an orally or parenterally administered dose form. These include tablets, powders, chewable tablets, capsules, oral suspensions, suspensions, emulsions or fluids, children's formulations, enteral feeds, nutraceuticals, and functional foods.

The dose form may further include any additives routinely used in preparation of that dose form such as starch or polymeric binders, sweeteners, coloring agents, emulsifiers, coatings and the like. Other suitable additives will be readily apparent to those skilled in the art.

In one embodiment, the dose form has an enteric coating as disclosed in international patent application PCT/AU01/01206, incorporated herein by reference.

In another embodiment, the dose form is a topical formulation as disclosed in international patent application PCT/AU02/01003, incorporated herein by reference.

The dose form may contain other pharmaceutical compounds which do not antagonise the activity of the phosphate derivatives of electron transfer agents. The other pharmaceutical compound may be administered before, with or after the one or more phosphate derivatives of one or more electron transfer agents. Preferably, the other pharmaceutical compounds are hypercholesterolaemic compounds. More preferably, the other pharmaceutical compounds are selected from the group consisting of statins, phosphate derivatives of statins and mixtures thereof. Examples of appropriate statins include provastatin, lovastatin and atorvastatin and phosphates thereof.

### Brief Description of Drawings

Figure 1. Effect of tocopherol on cell proliferation
Figure 2. Effect of tocopheryl phosphate mixture on cell proliferation
Figure 3. Effect of tocopheryl phosphate on cell proliferation
Figure 4. Inhibition of cell proliferation by various compositions - adhered cell counts
Figure 5: Inhibition of cell proliferation by various compositions - MTS
Figure 6: FACS with THP-1 monocytes and anti-CD36-FITC
Figure 7: Binding and uptake of ocLDL-DiO in THP-1 monocytes
Figure 8: Growth inhibition of THP-1 monocytes.

### Examples

The invention will now be further illustrated and explained in the following non-limiting examples.

### Example 1

This example investigated the effect of tocopherol and tocopheryl phosphates on Rat Aortic Smooth Muscle Cell proliferation.

The Rat Aortic Smooth Muscle Cells (RASMC) used in proliferative studies are derived from the tunica intima and tunica media of healthy, fibrous plaque-free adult rat aorta. This cell line is an accepted model for the study of atherosclerosis, since increased arterial smooth muscle mass are found in the intima lesion of atherosclerosis. RASMC are cryopreserved at second passage and can be propagated at least 16 population doublings. RASMC respond to various factors by cell proliferation and hypertrophy, which are prominent indicators of atherosclerosis in vascular disease. RASMC are well suited for the study of large vessel smooth muscle cell growth and differentiation and serve as an *in vitro* model in correlation with live rat models.

### Materials

- 6 well plates (Cell Counts)
- 96 well plates (MTT Assay)
- DMEM/F12 Medium - GIBCO/Life Technologies
- Fetal Bovine Serum (serum) -
- Rat Aortic Smooth Muscle Cells (RASMCs) p: 4 Cell Applications, Inc.
- Gentamicin - GIBCO/Life Technologies
- Cell Titer 96 Aqueous One Solution (MTT) - Promega
- Ethanol (EtOH), 1/1000
- Tocopherol (0.25, 0.5, 1, 5, 10, 20, 50, 100µM)
- Tocopherol phosphate (mixture of TP and T2P) (0.25, 0.5, 1, 5, 10, 20, 50, 100µM)

### Smooth Muscle Cell Proliferation - Cell Counts

Rat Aortic Smooth Muscle Cells (RASMC) were seeded in growth medium (basal medium + 10% FBS) into 6 well plates (50,000 cells/well). After 24 h, cells were washed twice with Hanks Buffered Salt Solution and serum depleted media (basal medium + 0.2% FBS) was added to each well. Cells were serum starved for 48 h. Treatments were then prepared in growth medium and added to each well (3 ml/well). Each treatment was conducted in triplicate. The effect of tocopherol and tocopherol phosphate on smooth muscle cell proliferation was tested at eight concentrations: 0.25, 0.5, 1, 5, 10, 20, 50 and 100 µM. Control treatments included: growth medium and growth medium + vehicle (EtOH, 1/1000). After a 72 h incubation period at 37°C, 5% CO₂, cells were counted.

### Results

Cell proliferation was assessed and quantified by cell counting. Cells were counted after 48 h starvation in basal medium supplemented with 0.2% serum before the addition of test compounds. The average cell count of triplicate wells was ∼60,000. This number exceeds the number of cells seeded prior to starvation (50,000). Therefore the cells were determined to be viable and treatments were added to each well as described previously.

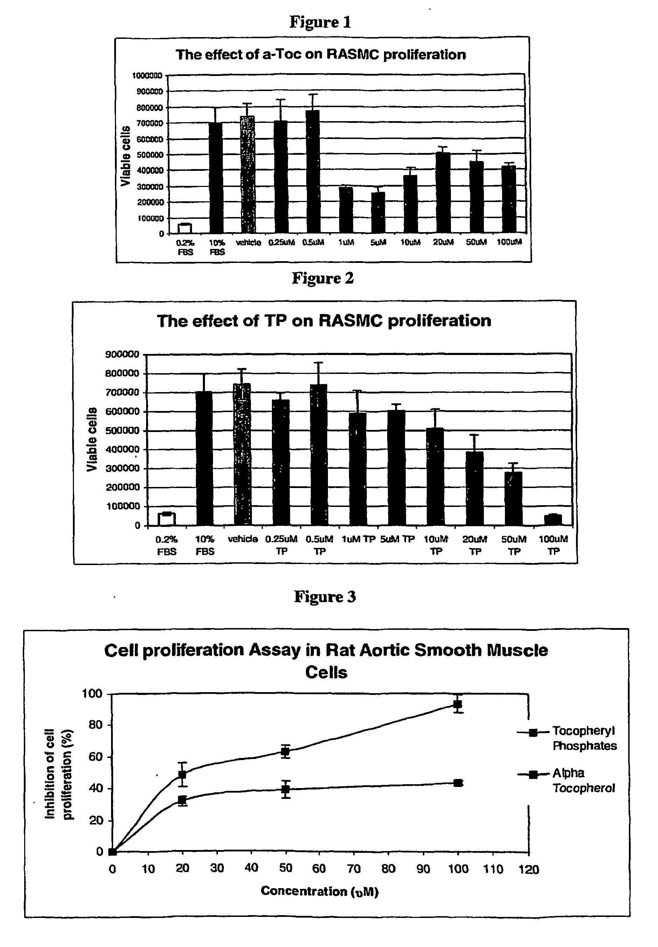

Cells were treated with compounds for 72h and then counted. Figure 1 and Figure 2 set out the results obtained for tocopherol and tocopherol phosphate, respectively. Under the conditions tested, the vehicle in which the test compounds were diluted, EtOH (1/1000), did not affect cellular proliferation. Tocopherol inhibited only to some degree cellular proliferation at 1, 5, 10, 20, 50 and 100µM. Tocopherol phosphate, however, inhibited cellular proliferation in a dose dependent manner at 1, 5, 10, 20, 50 and 100µM. 100% inhibition of cellular proliferation was observed at 100µM tocopherol phosphate (Figure 3).

### Discussion

The results demonstrate that the tocopheryl phosphate mixture can inhibit excessive cellular proliferation at 5µM, 20µM, 50 µM and 100 µM in a dose dependant manner. Importantly, complete inhibition of new cell formation was achieved at 50 µM.

α-Tocopherol treatment partially inhibited cellular proliferation at 1 µM, 5 µM and 10 µM but the higher doses did not completely reduce proliferation. Optimum inhibition of proliferation plateaued around 60% which is in agreement with published literature. This would make α-tocopherol unreliable and thus not suitable for use in treating atherosclerosis. On this basis and according to recently published literature there is no rational for using α-tocopherol for atherosclerosis.

Despite α-tocopherol working at a lower dose to provide partial inhibition, tocopheryl phosphate is clearly a more potent anti-proliferative agent as it was capable of achieving 100% inhibition of cell proliferation. Further, inhibition of excessive cellular proliferation occurred in a dose dependant manner indicating that the tocopheryl phosphate mixture is a more reliable and predictable therapy making it suitable for use in treating atherosclerosis.

In summary, the tocopheryl phosphates mixture acts in a dose dependant manner and thus provides more reliable and effective inhibition of excessive cellular proliferation than α-tocopherol. More importantly, the tocopheryl phosphate mixture achieved 100% inhibition of cell proliferation at 50 and 100 µM. This indicated that the tocopheryl phosphate mixture could be used as a direct treatment for atherosclerosis, as it is surprisingly capable of preventing early initiation steps of smooth muscle cell proliferation in a predictable manner.

### Example 2

This example assesses the anti-proliferative activity of α-tocopheryl phosphate (TP), di-tocopheryl phosphate (T2P), the TP/T2P mixture and α-tocopherol using two types of cell counting assays: adhered cell counts and MTS assay.

The MTS proliferation assay was conducted to further support and compliment the adhered cell counts assay. The MTS assay is a well established method for the assessment of cellular proliferation which takes into account the viable cells that are adhered to the plate (as in adhered cell counts) and those that may become detached and float in the media during the course of the experiment (which would be missed in adhered cell counts).

### Materials

6 well plates (Cell Counts)
96 well plates (MTS Assay)
DMEM/F12 Medium-GIBCO/Life Technologies
Fetal Bovine Serum (serum)
Rat Aortic Smooth Muscle Cells (RASMC) p: 4 Cell Applications, Inc.
Gentamicin - GIBCO/Life Technologies
Cell Titer 96 Aqueous One Solution (MIT) - Promega
Ethanol (EtOH). 1/1000
Tocopherol SIGMA (0, 20, 50, 100µM)
TP/T2P mixture (80%:20%) (0, 20, 50, 100µM)
Tocopheryl phosphate pure (0, 20. 50, 100µM)
Di-tocopheril phosphate pure (0, 20, 50, 100µM)

### Results

There was no statistical difference between the media alone and media plus vehicle. All of the data has been derived from % differences from vehicle controls (i.e., vehicle control is 0% on graph).

### Study 1. Counting adhered cells

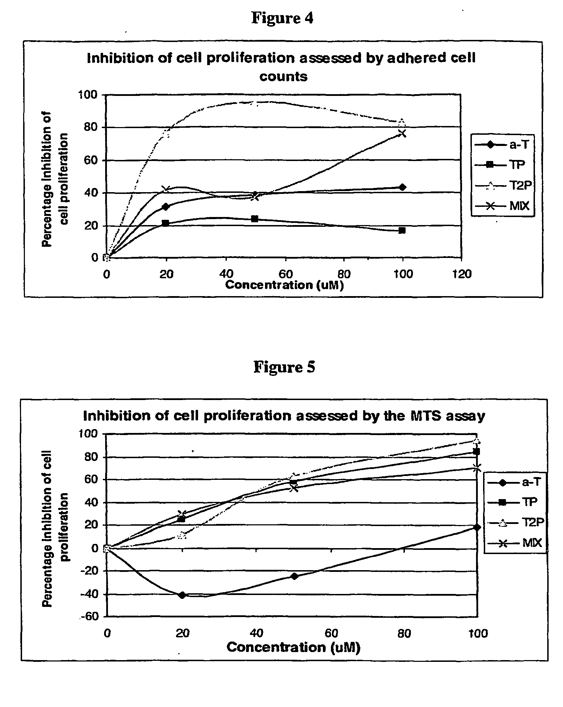

In this study cells remaining on the bottom of the plate were counted and anti-proliferative activity was assessed based on the number of viable cells that remained adhered to the plate. Results suggest that both T2P and the TP/T2P mixture were both potent anti-proliferative agents causing maximum (85-90%) inhibition of smooth muscle cell proliferation, whereas TP did not inhibit smooth muscle cell proliferation in this assay (Figure 4).

### Study 2. MTS assay

The results from this study demonstrate that once again T2P and the TP/T2P mixture are able to inhibit smooth muscle cell proliferation by up to 85-90% at a 100 µM concentration (Figure 5). Interestingly, pure TP was also capable of inhibiting smooth muscle cell proliferation (up to 85%) at 100 µM. This suggests that the mechanism for the TP anti-proliferative effects is different to that for both T2P alone and the TP/T2P mixture.

### Conclusions

These findings suggest that TP, T2P and the TP/T2P mixture are all potent anti-proliferative agents when compared with α-tocopherol. TP is as active as T2P in inhibiting smooth muscle cell proliferation. However, TP appears to exert its anti-proliferative activity in a different manner to T2P, the TP/T2P mixture and α-tocopherol.

### Example 3

The aim of this study was to compare the effects of a TP/T2P mixture with tocopherol on the expression of CD36, the uptake of oxidised-LDL (oxLDL) and the growth of human TBP-1 monocytes *in vitro.*

### Methods

*Cell Culture:* Tocopherol and the TP/T2P mixture were each dissolved in ethanol, and the concentrations of the stock solutions were confirmed spectrophotometrically. Monocytes (THP-1) were grown in RPMI/10% FCS.

*Labeling oxLDL* OxLDLs (90% to 100% oxidation) were purchased from Intracell Corp. Small amounts of LDL were oxidized with CuSO₄ (20 mmol/L) at 37°C for 18 to 22 hours. LDL oxidation was confirmed by the formation of a characteristic smear band on an agarose gel. Labeling of oxLDL was done basically as previously described. OxLDLs were incubated at 37°C with DiO (Molecular Probes) in lipoprotein-deficient serum (Sigma) for 15 hours. The labeled oxLDLs (oxLDL-DiO) were purified by ultracentrifugation over a KBr gradient and dialyzed against several changes of saline-EDTA (1.5 mol/L NaCl-0.01% EDTA) for 6 hours.

*Uptake of oxLDL:* Uptake of oxLDL was studied with fluorescence-activated cell sorting (FACS). For FACS, the cells were pretreated for 16 hours with 50 µM tocopherol, tocopheryl phosphates, or ethanol solvent (control) and then incubated with oxLDL-DiO (5 µg/mL medium) for 6 hours. For competition experiments, the cells were incubated with monoclonal anti-CD36 antibody (60 µg/5 mL DMEM) (Ancell), with an unspecific isotype-matched antibody (mouse IgM, Ancell), or with unlabeled oxLDL (100 µg/5 mL DMEM) (Intracell Corp). Thereafter, the cells were washed 3 times with PBS and twice with PBS-3 mg/mL BSA and then were detached with trypsin (0.25% trypsin, 0.03% EDTA). The cells were harvester with DMEM/10% FCS, centrifuged, washed twice with PBS, and then fixed with 4% paraformaldehyde in PBS. FACS was performed with a FACScan (Becton-Dickinson). Data were calculated by subtracting the cell autofluorescence from the fluorescence of the treated samples.

*Thin Layer. Chromatography* The eluent used was chloroform / hexane (1:1 v/v) and the development time was 20 min. Detection was by UV at 254 nm

### Results and discussion

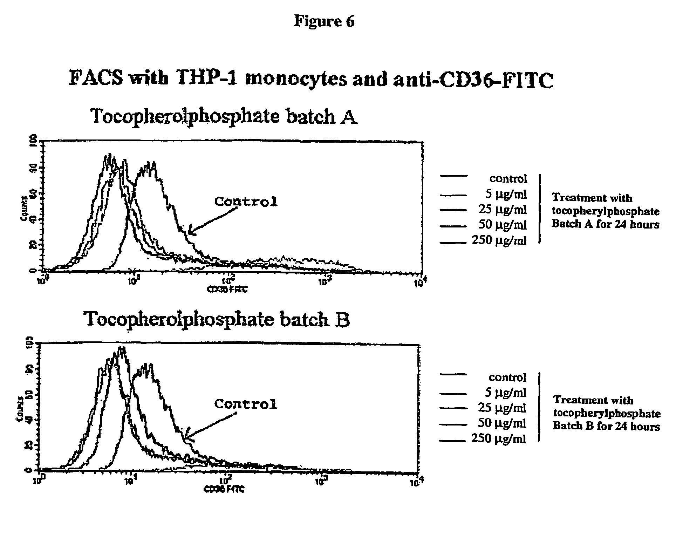

### Surface Expression of CD36 Scavenger Receptor (Figure 6):

Figure 6 represents the change in cell numbers over time as indicated by the number of cells which have taken up the fluorescent antibody. The peaks for the TP/T2P mixture are shifted to the left from the peak for the control (tocopherol) demonstrating that fewer cells have taken up the antibody and therefore there is less CD36 receptor expression.

Treatment of THP-1 monocytes (human origin) with as little as 5 µg/ml of TP/T2P mixture resulted in a substantial reduction of CD36 expression. The control cells (treated with tocopherol) expressed large amounts of CD36 receptors as indicated by the strong fluorescent labelling with anti-CD36 fluorescent antibodies. 5µg of TP/T2P mixture was capable of suppressing CD36 receptor expression as indicated by its large shift to the left. Note that the scale is logarithmic.

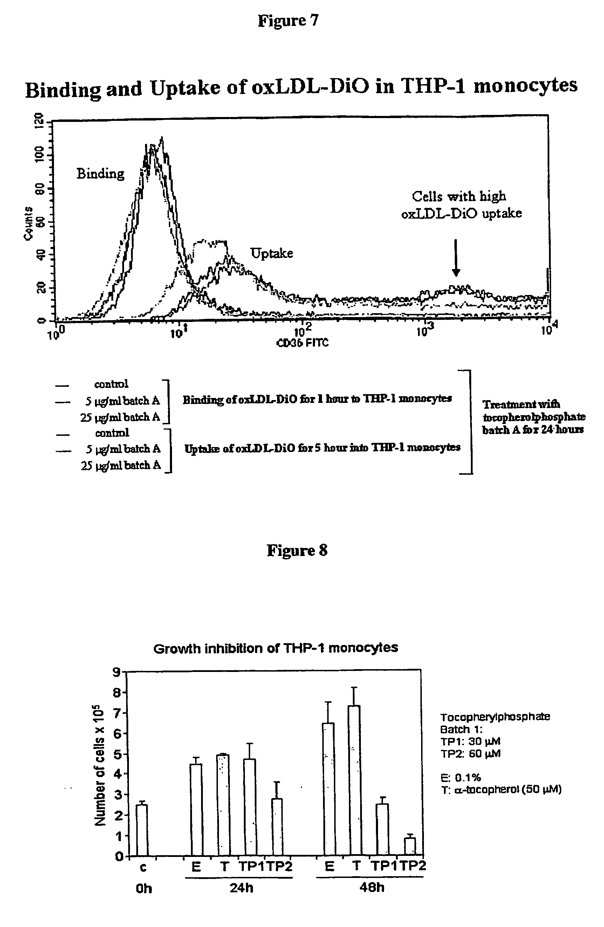

### Binding and Uptake of oxLDL-DiO (Figure 7):

Figure 7 represents the change in cell numbers over time as indicated by the number of cells which have taken up the labelled oxLDL. The curves are all similar which demonstrates that the TP/T2P mixture at concentrations of just 5 and 25 µg/ml achieved the same effect as the tocopherol (control) at 22µg/ml (50 µM). The arrow highlights the fact that the TP/T2P mixture at 25 µg/ml achieved a significantly larger reduction of oxLDL uptake.

TP/T2P was tested with human THP-1 monocytes. Binding of oxLDL-DiO was weakly inhibited at 5 µg/ml, and more at 25 µg/ml. Uptake of oxLDL-DiO was already inhibited at 5 µg/ml and much more at 25 µg/ml.

OxLDL-DiO uptake signal (median of the peak) was reduced by 33 % in cells treated with tocopherol at 50 µM concentration. The same effect in cells treated with the TP/T2P mixture was obtained at a concentration of less than 10 µM. It can be inferred that inhibition of CD36 expression by tocopherol leads to reduced CD36-mediated oxLDL uptake and that the same effect is obtained with the TP/T2P mixture at lower concentrations. A cell population (indicated by the arrow) with high uptake capacity for oxLDL-DiO was highly inhibited by the TP/T2P mixtures.

Growth Inhibition by TP/T2P Mixture of THP-1 Monocytes: Monocyte proliferation is an important event in the onset and progression of atherosclerosis. Figure 8 indicates that tocopherol (T) is not able to inhibit this proliferation relative to the ethanol control (E). On the other hand, the TP/T2P mixture inhibited proliferation at 30 µM (TP1) and 60 µM (TP2), especially after 48 h treatment.

### Conclusion

This example shows that the CD36 scavenger receptor is significantly inhibited by the TP/T2P mixture. Such an inhibition of CD36 expression leads to a diminution of oxidised LDL uptake. Moreover, the TP/T2P mixture inhibits the proliferation of monocytes. This event appears to be unique for the TP/T2P mixture and was not achieved using tocopherol. The results obtained in relation to tocopherol agree with those previously published regarding tocopherol.
- The TP/T2P mixture was shown to significantly decrease the expression of CD36 receptor in human monocytes. In general, the results showed that the potency of the TP/T2P mixture is 5-10 fold higher than that of tocopherol.
- The binding and uptake of oxidised LDL by monocytes was significantly better inhibited by TP/T2P when compared to tocopherol. The degree of inhibition that was seen with 10µM TP/T2P, required 50µM tocopherol (i.e., five times less TP/T2P mixture was required).
- TP/T2P significantly inhibited the proliferation of human monocyte cells (more than 90% inhibition with TP/T2P but no inhibition of proliferation with tocopherol).

The study demonstrated TP/T2P was more effective than α-tocopherol at reducing uptake of oxidized cholesterol, inhibiting CD 36 receptor expression which subsequently reduced oxLDL uptake and inhibiting the proliferation and migration of monocytes.

More importantly, TP/TZP worked in a dose dependant manner and provided more significant reduction of effect than tocopherol.

The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

## Claims

1. A pharmaceutical composition for use in inhibiting plaque formation in the vascular system, the composition comprising an effective amount of one or more phosphate derivatives of one or more electron transfer agents selected from the group consisting of mono-tocopheryl phosphate, di-tocopheryl phosphate and mixtures thereof.

2. The pharmaceutical composition according to claim 1 wherein the electron transfer agent is selected from the group consisting of α-tocopherol, β-tocopherol, δ-tocopherol, γ-tocopherol and mixtures thereof.

3. The pharmaceutical composition according to claim 2 wherein the electron transfer agent is α-tocopherol.

4. A dose form comprising the pharmaceutical composition of any one of claims 1 to 3.

5. The dose form according to claim 4 further comprising one or more other pharmaceutical compounds.

6. The dose form according to claim 5 wherein the one or more other pharmaceutical compounds are selected from the group consisting of statins, phosphate derivatives of statins and mixtures thereof.

7. Use of an effective amount of one or more phosphate derivates of one or more electron transfer agents, selected from the group consisting of mono-tocopheryl phosphate, di-tocopheryl phosphate and mixtures thereof, together with a suitable carrier or diluent in the manufacture of a medicament for inhibiting plaque formation in the vascular system.

8. The use according to claim 7 wherein the electron transfer agent is selected from the group consisting of α-tocopherol, β-iocopherol, δ-tocopherol, γ-tocopherol and mixtures thereof.

9. The use according to claim 7 wherein the effective amount is an amount which is 0.1 to 10 times the average plasma concentration of α-tocopherol.

10. The use according to claim 9 wherein the effective amount is an amount which is 2 to 3 times the average plasma concentration of α-tocopherol.

11. The use according to claim 7 wherein the one or more phosphate derivatives of electron transfer agents are in the form of one or more complexes of phosphate derivatives of electron transfer agents.

12. The use according to claim 8 wherein the electron transfer agent is α-tocopherol.

13. The use according to claim 12 wherein the one or more phosphate derivatives of one or more electron transfer agents are a mixture of mono-tocopheryl phosphate and di-tocopheryl phosphate.

## Patentansprüche

1. Eine pharmazeutische Zusammensetzung zur Verwendung zur Inhibierung der Bildung von Ablagerungen im Gefäßsystem, wobei die Zusammensetzung eine wirksame Menge eines oder mehrerer Phosphatderivate eines oder mehrerer Elektronentransfermittel, die aus der Gruppe bestehend aus mono-Tocopherylphosphat, di-Tocopherylphosphat und Gemischen davon ausgewählt sind, umfasst.

2. Die pharmazeutische Zusammensetzung gemäß Anspruch 1, wobei das Elektronentransfermittel aus der Gruppe bestehend aus α-Tocopherol, β-Tocopherol, δ-Tocopherol, γ-Tocopherol und Gemischen davon ausgewählt ist.

3. Die pharmazeutische Zusammensetzung gemäß Anspruch 2, wobei das Elektronentransfermittel α-Tocopherol ist.

4. Eine Dosierungsform, die die pharmazeutische Zusammensetzung gemäß einem der Ansprüche 1 bis 3 umfasst.

5. Die Dosierungsform gemäß Anspruch 4, die ferner eine oder mehrere weitere pharmazeutische Verbindungen umfasst.

6. Die Dosierungsform gemäß Anspruch 5, wobei die eine oder die mehreren weiteren pharmazeutischen Verbindungen aus der Gruppe bestehend aus Statinen, Phosphatderivaten von Statinen und Gemischen davon ausgewählt sind.

7. Verwendung einer wirksamen Menge eines oder mehrerer Phosphatderivate eines oder mehrerer Elektronentransfermittel, die aus der Gruppe bestehend aus mono-Tocopherylphosphat, di-Tocopherylphosphat und Gemischen davon, ausgewählt sind, zusammen mit einem geeigneten Träger oder Verdünnungsmittel bei der Herstellung eines Medikaments zur Inhibierung der Bildung von Ablagerungen im Gefäßsystem.

8. Die Verwendung gemäß Anspruch 7, wobei das Elektronentransfermittel aus der Gruppe bestehend aus α-Tocopherol, β-tocopherol, δ-Tocopherol, γ-Tocopherol und Gemischen davon ausgewählt ist.

9. Die Verwendung gemäß Anspruch 7, wobei die wirksame Menge eine Menge ist, welche das 0,1 bis 10-fache der mittleren Plasmakonzentration von α-Tocopherol ist.

10. Die Verwendung gemäß Anspruch 9, wobei die wirksame Menge eine Menge ist, welche das 2 bis 3-fache der mittleren Plasmakonzentration von α-Tocopherol ist.

11. Die Verwendung gemäß Anspruch 7, wobei das eine oder die mehreren Phosphatderivate von Elektronentransfermitteln in Form eines oder mehrerer Komplexe von Phosphatderivaten von Elektronentransfermitteln vorliegen.

12. Die Verwendung gemäß Anspruch 8, wobei das Elektronentransfermittel α-Tocopherol ist.

13. Die Verwendung gemäß Anspruch 12, wobei das eine oder die mehreren Phosphatderivate eines oder mehrerer Elektronentransfermittel ein Gemisch von mono-Tocopherylphosphat und di-Tocopherylphosphat sind.

## Revendications

1. Composition pharmaceutique pour une utilisation dans l'inhibition de la formation de plaques dans le système vasculaire, la composition comprenant une quantité efficace d'un ou plusieurs dérivés phosphatés d'un ou plusieurs agents de transfert d'électrons choisis dans le groupe constitué par le phosphate de mono-tocophéryle, le phosphate de di-tocophéryle et des mélanges de ceux-ci.

2. Composition pharmaceutique selon la revendication 1, dans laquelle l'agent de transfert d'électrons est choisi dans le groupe constitué par l'α-tocophérol, le β-tocophérol, le δ-tocophérol, le γ-tocophérol et des mélanges de ceux-ci.

3. Composition pharmaceutique selon la revendication 2, dans laquelle l'agent de transfert d'électrons est l'α-tocophérol.

4. Forme posologique comprenant la composition pharmaceutique selon l'une quelconque des revendications 1 à 3.

5. Forme posologique selon la revendication 4, comprenant en outre un ou plusieurs autres composés pharmaceutiques.

6. Forme posologique selon la revendication 5, dans laquelle le ou les autres composés pharmaceutiques sont choisis dans le groupe constitué par les statines, les dérivés phosphatés des statines et des mélanges de ceux-ci.

7. Utilisation d'une quantité efficace d'un ou plusieurs dérivés phosphatés d'un ou plusieurs agents de transfert d'électrons, choisis dans le groupe constitué par le phosphate de mono-tocophéryle, le phosphate de di-tocophéryle et des mélanges de ceux-ci, avec un excipient ou un diluant approprié, dans la fabrication d'un médicament destiné à inhiber la formation de plaques dans le système vasculaire.

8. Utilisation selon la revendication 7, dans laquelle l'agent de transfert d'électrons est choisi dans le groupe constitué par l'α-tocophérol, le β-tocophérol, le δ-tocophérol, le γ-tocophérol et des mélanges de ceux-ci.

9. Utilisation selon la revendication 7, dans laquelle la quantité efficace est une quantité qui est 0,1 à 10 fois supérieure à la concentration plasmatique moyenne de l'α-tocophérol.

10. Utilisation selon la revendication 9, dans laquelle la quantité efficace est une quantité qui est 2 à 3 fois supérieure à la concentration plasmatique moyenne de l'α-tocophérol.

11. Utilisation selon la revendication 7, dans laquelle le ou les dérivés phosphatés des agents de transfert d'électrons sont sous la forme d'un ou de plusieurs complexes de dérivés phosphatés des agents de transfert d'électrons.

12. Utilisation selon la revendication 8, dans laquelle l'agent de transfert d'électrons est l'α-tocophérol.

13. Utilisation selon la revendication 12, dans laquelle le ou les dérivés phosphatés d'un ou plusieurs agents de transfert d'électrons sont un mélange de phosphate de mono-tocophéryle et de phosphate de di-tocophéryle.
